# EUROPEAN PATENT SPECIFICATION

(11) **EP 1 000 177 B1**
(45) Date of publication and mention of the grant of the patent: **25.04.2007**
(21) Application number: 98938310.4
(22) Date of filing: 31.07.1998
(51) Int. Cl.: C12Q 1/70, C07K 7/00, C07K 14/00, C07K 14/16, G01N 33/569

(54) **SYNTHETIC ANTIGENS FOR THE DETECTION OF ANTIBODIES HAVING IMMUNOREACTIVITY AGAINST HIV VIRUS**
SYNTHETISCHE ANTIGENE ZUM NACHWEIS VON ANTIKÖRPERN MIT IMMUNREAKTIVITÄT GEGEN HIV-VIRUS
ANTIGENES SYNTHETIQUES POUR LA DETECTION DES ANTICORPS IMMUNOREACTIFS AU VIRUS VIH

(30) Priority: 01.08.1997 US 904826
(43) Date of publication of application: 17.05.2000
(73) Proprietor: GENETIC SYSTEMS CORPORATION, Redmond, Washington 98052 (US)
(72) Inventor: COLEMAN, Patrick, F., Edmonds, WA 98026 (US); CHONG-DUG SU, Peter, Mercer Island, WA 98040 (US); MONJI, Nobuo, Seattle, WA 98115 (US); COLE, Carol-Ann, Seattle, WA 98112 (US); GOSHORN, Alice, Kamp, Seattle, WA 98177 (US)
(74) Representative: Gowshall, Jonathan Vallance
(86) International application number: PCT/US1998/016160
(87) International publication number: WO 1999/006599

(56) References cited:
- EP-A- 0 267 802
- RU-C- 2 043 411
- US-A- 4 629 783
- US-A- 5 075 211
- US-A- 5 206 136
- US-A- 5 439 792
- US-A- 5 580 739
- POLLET D E ET AL: "CONFIRMATION AND DIFFERENTIATION OF ANTIBODIES TO HUMAN IMMUNODEFICIENCY VIRUS 1 AND 2 WITH A STRIP-BASED ASSAY INCLUDING RECOMBINANT ANTIGENS AND SYNTHETIC PEPTIDES" CLINICAL CHEMISTRY, vol. 37, no. 10 PART 1, 1991, pages 1700-1707, XP001105753 ISSN: 0009-9147
- WAIN-HOBSON S ET AL: "NUCLEOTIDE SEQUENCE OF THE AIDS VIRUS, LAV" CELL, MIT PRESS, CAMBRIDGE, MA,, US, vol. 40, no. 1, 1985, pages 9-17, XP000608742 ISSN: 0092-8674
- STERNBERG M J ET AL: "Prediction of antigenic determinants and secondary structures of the major AIDS virus proteins." FEBS LETTERS. NETHERLANDS 29 JUN 1987, vol. 218, no. 2, 29 June 1987 (1987-06-29), pages 231-237, XP001105722 ISSN: 0014-5793
- SPRENGERS E D ET AL: "A MICRO-ELISA SYSTEM FOR THE DETECTION OF BOTH ANTI-HIV-1 AND ANTI-HIV-2 ANTIBODIES" JOURNAL OF IMMUNOLOGICAL METHODS, vol. 139, no. 1, 1991, pages 77-82, XP001105718 ISSN: 0022-1759
- DATABASE BIOSIS [Online] BIOSCIENCES INFORMATION SERVICE, PHILADELPHIA, PA, US; 1992 IVANOV V S ET AL: "STUDY OF THE ANTIGENIC STRUCTURE OF HUMAN IMMUNODEFICIENCY VIRUSES BY MEANS OF SYNTHETIC PEPTIDES" Database accession no. PREV199294121790 XP002213768 & BIOORGANICHESKAYA KHIMIYA, vol. 18, no. 6, 1992, pages 784-793, ISSN: 0132-3423
- DATABASE BIOSIS [Online] BIOSCIENCES INFORMATION SERVICE, PHILADELPHIA, PA, US; 1995 MONJI N ET AL: "Sensitive and specific HIV-1/HIV-2 EIA using env and pol synthetic peptides." Database accession no. PREV199698616393 XP001105703 & TRANSFUSION (BETHESDA), vol. 35, no. 10 SUPPL., 1995, page 28S 48th Annual Meeting of the American Association of Blood Banks;New Orleans, Louisiana, USA; November 11-15, 1995 ISSN: 0041-1132
- DATABASE BIOSIS [Online] BIOSCIENCES INFORMATION SERVICE, PHILADELPHIA, PA, US; 1995 MONJI NOBUO ET AL: "Specific and sensitive HIV-1/HIV-2 EIA using env and pol synthetic peptides." Database accession no. PREV199598347548 XP001105443 & CLINICAL CHEMISTRY, vol. 41, no. S6 PART 2, 1995, page S50 47th Annual Meeting of the American Association for Clinical Chemistry, Inc.;Anaheim, California, USA; July 16-20, 1995 ISSN: 0009-9147
- CLAVEL F ET AL: "Molecular cloning and polymorphism of the human immune deficiency virus type 2." NATURE. ENGLAND 1986 DEC 18-31, vol. 324, no. 6098, 18 December 1986 (1986-12-18), pages 691-695, XP000605290 ISSN: 0028-0836
- DATABASE SWISS-PROT [Online] Database accession no. P04586 XP002239588
- DATABASE SWISS-PROT [Online] Database accession no. P04585 XP002239589
- DATABASE SWISS-PROT [Online] Database accession no. P04584 XP002239590
- DATABASE EMBASE [Online] ELSEVIER SCIENCE PUBLISHERS, AMSTERDAM, NL; Database accession no. K02013.1 XP002239591
- FERNS R B ET AL: "MONOCLONAL ANTIBODIES DEFINE LINEAR AND CONFORMATIONAL EPITOPES OF HIV-1 POL GENE PRODUCTS" AIDS RESEARCH AND HUMAN RETROVIRUSES, NEW YORK, NY, US, vol. 7, no. 3, March 1991 (1991-03), pages 307-313, XP001011430 ISSN: 0889-2229
- MYERS R A ET AL: "IDENTIFYING HIV-2-SEROPOSITIVE INDIVIDUALS BY REEVALUATING HIV-1 INDETERMINATE SERA" JOURNAL OF ACQUIRED IMMUNE DEFICIENCY SYNDROMES, RAVEN PRESS, NEW YORK, NY, US, vol. 5, no. 4, April 1992 (1992-04), pages 417-423, XP001105714 ISSN: 0894-9255
- MODROW S ET AL: "CARRIER BOUND SYNTHETIC OLIGOPEPTIDES IN ELISA TEST SYSTEMS FOR DISTINCTION BETWEEN HIV-1 AND HIV-2 INFECTION" JOURNAL OF ACQUIRED IMMUNE DEFICIENCY SYNDROMES, RAVEN PRESS, NEW YORK, US, vol. 2, no. 2, 1 April 1989 (1989-04-01), pages 141-148, XP000578021
- HICKMAN R K ET AL: "DETECTION AND DIFFERENTIATION OF HIV-1 GROUP O SERA FROM HIV-1 GROUP M AND HIV-2 USING RECOMBINANT ANTIGENS AND PEPTIDES" JOURNAL OF VIROLOGICAL METHODS, AMSTERDAM, NL, vol. 72, no. 1, May 1998 (1998-05), pages 43-49, XP000866196 ISSN: 0166-0934
- RATNER et al., "Complete Nucleotide Sequence of the AIDS Virus, HTLV-III", NATURE, 24 January 1985, Vol. 313, pages 277-284, XP002916367
- GUYADER et al., "Genome Organization and Transactivation of the Human Immunodeficiency Virus Type 2", NATURE, 16 April 1987, Vol. 326, pages 662-669, XP002916368

## Description

### Field of the Invention

This invention relates to synthetic polypeptides, recombinant polypeptides and recombinant polynucleotide sequences encoding the same, useful for detecting antibodies associated with human immunodeficiency virus type 1 (HIV-1) and/or type 2 (HIV-2) (as used herein, "HIV" used without reference to the type shall mean either or both types), and particularly relates to synthetic polypeptides which mimic antigenic epitopes of the gene products of the HIV polymerase region.

### Background of the Invention

Human immunodeficiency virus type 1 and 2 (HIV-1 and HIV-2) are known to cause acquired immune deficiency syndrome (AIDS). Both viruses apparently exhibit similar modes of transmission. HIV-1 and HIV-2 were both isolated in the early 1980's from African AIDS patients. Since then, cases have been found in most countries of the world. Because the HIV viruses exhibit rapid genetic drift, widely divergent strains are emerging. Thus, detection and treatment of variant strains has proven to be challenging and difficult.

Individuals with antibodies reactive with HIV-1 and/or HIV-2 are determined by immunoassays of the conventional sandwich ELISA format. These assays are comprised of an immobilized viral antigen, that may be comprised of viral lysate, retrovirus proteins or natural or synthetic polypeptides, that is contacted with blood or serum components suspected of containing HIV antibodies. While the existing commercial tests appear to have significantly diminished the transmission of HIV virus in blood products, each test configuration may have some disadvantages.

The possible disadvantages of viral lysate tests include: the need to grow and handle large quantities of live infectious virus; the possibility that the live virus might be incorporated into test materials; the variable nature of the resulting viral lysate; and the substantial number of false positive and false negative results that require additional confirmatory testing. These disadvantages may also be associated with the use of isolated viral proteins as antigens.

The use of synthetic polypeptides, (as used herein, "synthetic polypeptide" and "polypeptide" shall mean any one, all or a combination of the following: synthetic polypeptide, recombinant polypeptide, or polypeptide) which can be engineered to immunologically mimic antigenic epitopes of the HIV viruses, may avoid some of the above-mentioned disadvantages. One area of concern with the use of synthetic polypeptides (less than or equal to 60 residues in length) in diagnostic assays is the consideration that viral antigenic drift could result in the failure to detect HIV-1 or HIV-2 infected sera using these assays, presumably due to the limited presentation of viral epitopes. One method of guarding against such an occurrence is to include polypeptides from different immunodominant regions of the viral genome. Thus, synthetic polypeptides that immunologically mimic immunodominant regions of the HIV-1 and HIV-2 pol gene products are important additions to the already described polypeptides that mimic the env, gag, and pol proteins of HIV-1 and HIV-2. U.S. Patent Nos. 4,629,783 and 5,075,211 describe synthetic polypeptides that mimic antigenic determinants of HIV-1. Cosand U.S. Patent 5,075,211 describes synthetic polypeptides that immunologically mimic antigenic epitopes of HIV-1 proteins from the pol region, including two polypeptides which are similar to the polypeptides of this invention. In blood screening assays, the greater the immunoreactivity of the antigens used in the assay method, the less likely antibodies to a new variant or subtype of HIV-1 or HIV-2, present in a patient's sample, will be left undetected.

U.S. Patent 5,306,466 describes an "HIV-3 retrovirus" which was initially believed to be separate and distinct from HIV-1 and HIV-2. Researchers have since determined that the HIV-3 retrovirus is merely a particular subtype of HIV-1, now referred to as subtype O, or Group O (R. De Leys, et. al., J.Virol.: 1207-1216 (1990); L.G Gürtler, et. al., J.Virol.:1581-1585 (1994)).

By comparing various HIV-1 isolates researchers have shown that some regions of the genome are highly variable while others are reasonably well conserved. Similar polymorphisms have also been observed for HIV-2.

Despite the apparent similarities in disease state and transmission of HIV-1 and HIV-2 viruses, the virus types have been differentiated based on their genetic divergence. Based on genetic analysis viral isolates can be grouped according to their genetic homology to previous isolates. Today, HIV-1 and HIV-2 form the two main branches of the HIV genetic tree. DNA hybridization studies suggest that, while regions of extensive homology exist between HIV-1 and HIV-2, other regions seem very divergent. (Clavel et al, Science 233: 343 (1986)). In fact, HIV-2 has been shown to have, overall, only about 40% homology with HIV-1, and studies have shown little immunological cross reactivity between the envelope glycoproteins. The limited serologic cross reactivity between these viruses makes screening assays based on HIV-1 antigens insufficient for screening or diagnosis of HIV-2 infection in human sera.

### SUMMARY OF THE INVENTION

Polypeptide sequences capable of mimicking immunodominant regions of HIV-1 or HIV-2 proteins, encoded in the polymerase region, have been identified. These synthetic polypeptides, recombinant polypeptides and recombinant polynucleotide sequences encoding the same, are useful in the preparation of reagents for the screening of blood and blood products for exposure to HIV viruses. The polypeptides can be used in various specific binding assays for the detection of antibodies to HIV-1 and/or HIV-2 virus, for the detection of HIV-1 and/or HIV-2 antigens, or as immunogens in vaccine compositions.

### BRIEF DESCRIPTION OF THE DRAWINGS

FIG. 1 depicts a plasmid map of one of the recombinant constructs of the present invention, more specifically that of pGEX/*pol*23.
FIG. 2 depicts a plasmid map of one of the recombinant constructs of the present invention, more specifically that of pGEX/*pol*7.
FIG. 3 depicts a plasmid map of one of the recombinant constructs of the present invention, more specifically that of pQE/*pol*23.
FIG. 4 depicts a plasmid map of one of the recombinant constructs of the present invention, more specifically that of pQE/*pol*7.
FIG. 5 depicts a plasmid map of one of the recombinant constructs of the present invention, more specifically that of pThioHis/*pol*23.
FIG. 6 depicts a plasmid map of one of the recombinant constructs of the present invention, more specifically that of pThioHis/*pol*7.

### DETAILED DESCRIPTION OF THE SPECIFIC EMBODIMENTS

Novel polypeptides are provided that immunologically mimic proteins encoded by the HIV-1 or HIV-2 retroviruses, respectively, particularly proteins encoded in the polymerase region of the viral genome. Each polypeptide of the invention may be modified by introducing conservative or non-conservative substitutions into the polypeptide, usually fewer than 20 number percent, and more usually fewer than 10 number percent of the amino acids being exchanged. In those situations where regions are found to be structurally polymorphic, it may be desirable to vary one or more particular amino acid to more effectively mimic the differing epitopes of the different retroviral strains. In many instances to provide chemical stability, methionine may be replaced by norleucine (Nor).

One particularly useful means of choosing appropriate amino acid substitutions in a polypeptide of the invention would be a substitution which occurs naturally in one or more isolates of the virus.

In general, the term "polypeptide" or "peptide" used herein shall mean a chain of amino acid molecules possessing biological activity. The terms do not relate to a product of any specific length.

It should be understood that the polypeptide employed in the subject invention need not be identical to any particular HIV-1 or HIV-2 polypeptide sequence, so long as the subject compound is able to immunologically mimic an epitope of the pol region of at least one of the strains of the HIV-1 or HIV-2 retrovirus. Therefore, the subject polypeptide may be modified to include various changes, as mentioned above, such as insertions, deletions, and substitutions, either conservative or non-conservative, where such changes might provide for certain advantages in their use. By conservative substitutions it is intended substitutions within groups such as gly, ala; val, ile, leu; asp, glu; asn, gln; ser, thr; lys, arg; phe, tyr; and nor, met. Usually, the sequence will not differ by more than 20% from the sequence of at least one strain of an HIV-1 or HIV-2 retrovirus except where additional amino acids may be added at either terminus for the purpose of providing an "arm" by which the polypeptide of this invention may be conveniently immobilized. The arms will usually be at least 1 amino acid and may be 50 or more amino acids, more often 1 to 10 amino acids, in length. A polypeptide in which the amino acid sequence is modified by the substitution, addition, or deletion of amino acid residues should retain substantially all of the immunological reactivity of the unmodified polypeptide, which may be conveniently measured by radioimmuno-precipitation, immunofluorescence, or enzyme-linked immunosorbant assays.

In addition, one or more amino acids may be added to the termini of an oligopeptide or polypeptide to provide for ease of linking polypeptides one to another, for coupling to a support or larger polypeptide, for modifying the physical or chemical properties of the polypeptide or oligopeptide, or the like.

Amino acids such as tyrosine, cysteine, lysine, glutamic or aspartic acid, or the like, may be introduced at the C- or N-terminus of the polypeptide to provide for a useful functionality for linking. Cysteine is particularly preferred to facilitate covalent coupling to other polypeptides or, for example, to form dimers by oxidation. To form polymers, it is preferred to have at least two cysteine residues present in the molecules being linked, preferably by utilizing cysteine residues added to the terminal portions of the polypeptides. Combinations of cysteine with intervening amino acid spacers are also useful. For example, two cysteine residues can be separated by one or more amino acid residue. Glycine residues are particularly useful and from one to three glycine residues may be employed between amino acids. Lysine residues have also been found to be useful as linkers and from one to three lysine residues may be used to couple the polypeptides to a solid phase alone or in combination with other amino acids.

In addition, the subject polypeptide sequences may differ from the natural sequence after being modified by terminal-NH₂ acylation, e.g. acetylation, or thioglycolic acid amidation, terminal-carboxy amidation, e.g. with ammonia or methylamine, to provide stability, increased hydrophobicity for linking or binding to a support or other molecule, or for polymerization.

The polypeptides disclosed and partially claimed herein that are derived from the polymerase region of HIV-1 are described below. The family of HIV-1 polypeptides are encoded by the genomic polynucleotide sequences (LAI or BRU isolate) encompassing base pairs (bp) 4448 through (bp) 4585 (numbering of Genbank HIVBRUCG; Accession K02013, BRU isolate) or is encoded in the pol open reading frame from about amino acid residue numbers 940 to about 985.

Polypeptide I (not in accordance with the claimed invention) has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another polypeptide disclosed herein, Polypeptide II, also designated BRU124EX, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

A polypeptide of the invention, Polypeptide III, also designated BRU124F1X, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another polypeptide of the invention, Polypeptide IV, also designated BRU124F3X, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

The polypeptides disclosed herein that are derived from the polymerase region of HIV-2 are described below. Polypeptide V, also designated ROD124E1, is encoded by the polynucleotide sequence of the HIV-2 genome encompassing base pairs (bp) 4694 through (bp) 4861 (numbering by Genbank HIV2ROD; Accession M15390, HIV-2ROD isolate) or is encoded in the pol open reading frame from about amino acid residue numbers 956 through 1001.

Polypeptide V has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another disclosed polypeptide, Polypeptide VI , also designated ROD 124EX, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another disclosed polypeptide, Polypeptide VII, also designated ROD124C2X, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another disclosed polypeptide, Polypeptide VIII , also designated ROD124C1X, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another disclosed polypeptide, Polypeptide IX, also designated ROD 123 C3X, has the following polypeptide sequence: wherein X is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support, Y is absent, Cys or one or more amino acids added to facilitate coupling; and Z is OH or NH₂.

Another disclosed polypeptide, Polypeptide X , also designated POL2A1, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another disclosed polypeptide, Polypeptide XI, also designated ROD124C5X, has the following polypeptide sequence: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Of particular interest is the use of the mercaptan group of cysteines or thioglycolic acids used for acylating terminal amino groups or the like for linking two of the polypeptides or oligopeptides or combinations thereof by a disulfide linkage or a longer linkage. To achieve this, compounds may be employed having bis-haloacetyl groups, nitroarylhalides, or the like where the reagents are specific for thio groups. Thus, the linking between the two mercapto groups of the different polypeptides or oligopeptides may be a single bond or a linking group of at least 2, usually at least 4, and not more than about 16, usually not more than about 14 carbon atoms.

Alternatively, the region of the viral genome coding for the polypeptide of the invention may be cloned by conventional recombinant DNA techniques and expressed. These techniques include PCR mediated cloning, as well as, synthesis of single strand polynucleotide strands encoding the polypeptide of interest. See generally, Maniatis et al, Molecular Cloning, A Laboratory Manual, CSH, Cold Spring Harbor Laboratory, 1982.

Fragments from a polynucleotide sequence may be employed for expression of polypeptide fragments, conservative base changes can be made, where the modified codon(s) code for the same amino acid(s), or non-conservative changes in the coding sequence may be made, where the resulting amino acid may be a conservative or non-conservative change in the amino acid sequence, which was discussed previously.

The coding sequence may be extended at either the 5'- or 3'-terminus or both termini to extend the polypeptide, while retaining its epitopic site(s). The extension may provide for an arm for linking, e.g., to a label, such as an enzyme, for joining this and other polypeptides together in the same chain, for providing antigenic activity, or the like.

For expression, the coding sequence will be provided with start and stop codons, promoter and terminator regions and usually a replication system to provide an expression vector for expression in a cellular host, e.g., prokaryotic or eukaryotic, bacterial, yeast, mammal, etc.

The DNA sequence by itself, fragments thereof, or larger sequences, usually at least 15 bases, preferably at least 18 bases, may be used as probes for detection of retroviral RNA or proviral DNA. Numerous techniques are described, such as the Grunstein-Hogness technique, Southern technique, Northern technique, dot-blot, improvements thereon, as well as other methodology. See, for example, WO 83/02277 and Berent et al, Biotechniques (1985) 3:208.

Conveniently, the polypeptide may be prepared as a fused protein, where the polypeptide may be the N- or C- terminus of the fused polypeptide. Commonly, the polypeptide of interest is expressed as a "translational fusion," meaning that the expressed protein will have its amino terminus attached to a "partner" protein that is easily expressed in *E. coli,* or other well known expression systems. The fusion also may include a purification tag or "handle."

One example of such a fusion system is the use of a portion of the glutathione S-transferase gene which encodes a protein that can be used in affinity-purification on glutathione-agarose resin. This fusion system is found in the commercially available pGEX plasmid (Pharmacia). A second example is the use of a portion of the dihydrofolate reductase gene as the fusion partner and a sequence encoding six histidine residues as the handle. The "His-tag" allows the resulting fusion protein to be purified by Ni-NTA (nickel) chromatography. This fusion system is found in the commercially available pQE42 plasmid (QIAGEN). Similarly, a portion of the *E. coli* thioredoxin gene having a "His-patch" consisting of three histidine residues engineered into the thioredoxin coding sequence allows purification of expressed proteins by Ni-NTA chromatography. This fusion system is found in the commercially available pThioHisA plasmid (Invitrogen).

A resulting fused protein could be used directly by itself as the reagent, or the subject polypeptide may be cleaved from all or a portion of the remaining sequence of the fused protein. With a polypeptide containing no internal methionines, by introducing a methionine at the fusion site, the polypeptide may be cleaved employing cyanogen bromide: Where there is an internal methionine, it would be necessary to provide for a proteolytic cleavage site, e.g., polylysine and/or arginine or combinations thereof. Alternatively, the internal methionine could be substituted by an amino acid such as leucine and an N-terminal methionine added for cyanogen bromide cleavage. A wide variety of proteases, including dipeptidases, are well known, and the appropriate processing signal could be introduced at the proper site. The processing signal may have tandem repeats so as to insure cleavage, since the presence of one or more extraneous amino acids will not interfere with the utility of the subject polypeptides.

Recombinant polynucleotide sequences encoding a number of polypeptides, derived from the polymerase region of HIV-1 are described below. The sequences are derived from the HIV-1 genomic polynucleotide sequences (LAI isolate) encompassing base pairs (bp) 2549 through (bp) 3139, and base pairs 4391 (bp) through (bp) 4648, as found in the HIV-1 viral lysate.

Polynucleotide Sequence I, also know as *pol*23, has the following sequence:

This sequence encodes the following amino acid sequence. (Without the intention of being bound by this teaching it is believed that the italicized amino acids represent particularly important residues to the immunoreactivity of the resulting polypeptide.)

A polynucleotide sequence of the invention, Polynucleotide Sequence II, also designated *pol*7, has the following sequence:

This sequence encodes the following amino acid sequence. (The italicized amino acids represent polypeptide BRU124F3X also disclosed herein.)

The subject polypeptides may be employed linked to a soluble macromolecular (e.g., not less than 5kDal) carrier. Conveniently, the carrier may be a poly(amino acid), either naturally occurring or synthetic, to which antibodies are unlikely to be encountered in human serum. Illustrative polypeptides include poly-L-lysine, bovine serum albumin, keyhole limpet hemocyanin, bovine gamma globulin, etc. The choice is primarily one of convenience and availability.

With such conjugates, there will be at least one molecule of at least one subject polypeptide per macromolecule and not more than about 1 per 0.5 kDal, usually not more than about 1 per 2 kDal of the macromolecule. One or more different polypeptides may be linked to the same macromolecule.

The manner of linking is conventional, employing such reagents as p-maleimidobenzoic acid, p-methyldithiobenzoic acid, maleic acid anhydride, succinic acid anhydride, glutaraldehyde, etc. The linkage may occur at the N-terminus, C-terminus or at a site intermediate to the ends of the molecule. The subject polypeptide may be derivatized by linking, may be linked while bound to a support, or the like.

The polypeptides of the invention may be used as reagents in assays to detect antibodies to HIV-1 or HIV-2 or antigens thereof. The polypeptides may be employed as labeled or unlabeled reagents depending upon their use (By label is intended a molecule which provides, directly or indirectly a detectable signal). Various labels may be employed, such as radionuclides, enzymes, fluorescers, chemiluminescers, enzyme substrates, cofactors or inhibitors, particles, e.g., magnetic particles, combinations of ligands and receptors, e.g., biotin and avidin, or the like. In addition the polypeptides may be modified in a variety of ways for binding to a surface, e.g., microwell plate, glass beads, chromatographic surface, e.g., paper, cellulose, silica gel, or the like. The particular manner by which the polypeptides are joined to another compound or surface is conventional and finds ample illustration in the literature. See, for example, U.S. Pat. Nos. 4,371,515; 4,487,715; and patents cited therein.

Various assay protocols may be employed for detecting the presence of either antibodies to retroviral proteins or retroviral proteins themselves. Of particular interest is using the polypeptide as the labeled reagent, where the label allows for a detectable signal, or binding the polypeptide, either directly or indirectly to a surface, where antibody or the polypeptide in the sample will become bound to the polypeptide on the surface. The presence of human antibody bound to the polypeptide can then be detected by employing a xenogeneic antibody specific for human immunoglobulin, normally both human IgM and IgG, or a labeled protein specific for immune complexes, e.g., RF factor or *S. aureus* Protein A.

Various heterogeneous protocols may be employed, either competitive or non-competitive. Polypeptide may be bound to a surface or support ("support") and labeled antibody allowed to compete with antibody in the sample for the limited amount of bound polypeptide. The amount of label bound to the support would be related to the amount of competitive antibody in the sample.

Xenogeneic anti-human antibody, e.g., antibodies to the Fc region of IgG and IgM (immunoglobulins), could be bound to a support. The sample would be contacted with the immunoglobulins and labeled polypeptide, whereby the amount of labeled polypeptide bound to the support would be indicative of the presence of the cognate antibodies.

Alternatively, homogeneous assays can be employed where the polypeptide is bound to an enzyme, fluorescer, or other label, where the binding of antibody to the polypeptide results in being able to discriminate between the label involved with a specific binding pair complex and label which is not involved in the complex. For assays involving such techniques, see for example U.S. Patent Nos. 3,817,837; 3,850,752; 3,901,654; 3,935,074; 3,984,533; 3,996,345; 4,034,074; and 4,098,876.

As an illustration of the subject invention, the subject polypeptide may be conjugated to a fluorescent molecule, such as fluorescein, rhodamine or umbelliferone. In this assay the fluorescence polarization is different between complexed and uncomplexed polypeptide conjugate. Apparatuses are available for measuring changes in fluorescence polarization, e.g., TDx supplied by Abbott Laboratories, Chicago, Illinois.

Illustrative of an assay technique is the use of a sample container, e.g. microwell plate wells, where the subject polypeptide or conjugates thereof are adhered to the container bottom and/or walls either covalently or noncovalently. The sample, normally human blood or serum diluted in appropriately buffered medium, is added to the container and a sufficient time allowed for complex formation between the polypeptide(s) and any cognate antibodies in the sample. The supernatant is removed and the container washed to remove nonspecifically bound proteins.

A labeled specific binding protein which specifically binds to the complex is employed for detection. To the container may be added xenogeneic antisera to human immunoglobulin, particularly anti-(human IgM and IgG) in an appropriately buffered medium. The xenogeneic antisera will normally be labeled with a detectable label, e.g., horseradish peroxidase. The label may then be detected. For example, with an enzyme, after removal of non-specifically bound enzyme label, a developer solution is added. The developer solution will contain an enzyme substrate and possibly enzyme cofactors, chromogens, etc., which, upon reaction, provide a colored, fluorescent, or chemiluminescent product which may be detected colorimetrically, fluorimetrically, or by photon counting, respectively.

The polypeptide can be prepared in a wide variety of ways. The polypeptide, because of its relatively short length, may be synthesized in solution or on a solid support in accordance with known protocols. See, for example, Stewart and Young, Solid Phase polypeptide Synthesis, 2^{nd} ed., Pierce Chemical Co., 1984; and Tam et al, J. Am. Chem. Soc. (1983) 105:6442.

Alternatively, as discussed herein, recombinant DNA technology may be employed where a recombinant polynucleotide sequence may be prepared by employing single strands which code for the polypeptide or substantially complementary strands thereof. (See, for example, Maniatis, *supra*.)

Depending upon the nature of the assay, the physiological sample, e.g., saliva, blood, plasma, or serum, may be pretreated by dilution into an assay medium, which will usually be an aqueous buffered medium employing one of a variety of buffers, such as phosphate, tris, or the like. A preferred diluent is 5% w/v nonfat dry milk, .01% Proclin 300,.005% Antifoam A in 150mM sodium citrate. Usually the pH will be in the range of about 6 to 9. The sample will then be combined with the reagent in accordance with appropriate protocol and sufficient time allowed for binding. Where a heterogeneous system is used, usually the binding stages will be followed by washes to minimize nonspecific binding. At the end of the procedure, the label will be detected in accordance with conventional methods.

Besides the use of the subject polypeptide and its analogs in assays, the subject polypeptide may also find use by itself or in combination in vaccines. The polypeptides may be formulated in a convenient manner, generally at concentrations in the range of 1 ug to 20 mg/kg of host. Physiologically acceptable media may be used as carriers, such as sterile water, saline, phosphate buffered saline, and the like. Adjuvants may be employed, such as aluminum hydroxide gel, or the like. Administration may be by injection, e.g., intramuscularly, intraperitoneally, subcutaneously, intravenously, etc. Administration may be one or a plurality of times, usually at one to four week intervals.

The immunoreactivity of the above-mentioned polypeptides I-IV, which immunologically mimic HIV-1 antigens, to eight known HIV-1 positive sera (i.e., GS91-[034, 037, 042, 046, 049, 052, 056, and 067], 11230, 11424, 11527, 11532 and 11535; all Western-blot all band positives) was examined, and the results are shown in Table 1A of Example 2. All of the polypeptides listed above were highly reactive to those samples.

Analogously, the immunoreactivity of each of the above-mentioned polypeptides, which immunologically mimic HIV-2 antigens, to five known HIV-2 positive sera (i.e., 92099,92100, P-83, P-84, and P-86; Western-blot all band positive) was examined, and the results are shown in Table 1B of Example 2. The glycoprotein (gp) polypeptide, 41-2-3GC (a nonglycosylated polypeptide that is the subject of co-pending U.S. application No. 08/268,388) was highly reactive to all five samples. All of the polypeptides listed above were reactive to at least two of those five samples. The most reactive polypeptides were ROD 124C2X and ROD 124C5X; they were reactive to all five samples.

### Example 1 - Synthesis of HIV Pol Polypeptides.

Series of HIV pol polypeptides were each synthesized by the sequential coupling of t-butyloxycarbonyl-protected amino acids onto 0.35 mmol p-methylbenzhydrylamine resin (Applied Biosystems Inc., Foster City, CA). Amino acid side chain protection was done by standard benzyl based groups. The tryptophan residue was protected by the formyl moiety. Completed polypeptides were deprotected and cleaved from the resin by the standard high HF procedure or the low-high HF procedure of Tam et al (J. Amer. Chem. Soc. 105:6442, 1983). The cleaved polypeptide was extracted from the resin in 50% acetic acid and subjected to Sephadex G-25 chromatography, using 20% acetic acid as a eluting solvent. Fractions containing polypeptide were pooled and lyophilized.

### Example 2 - Immunoreactivity of Pol Polypeptides:

Polypeptides of the invention were tested for immunological reactivity by ELISA as previously described in U.S. Patent No. 4,629,783. Briefly, stock solutions of polypeptides of the invention at 0.5mg/ml were prepared in 2M urea/5% acetic acid. Twelve milliters of 1.2% acetic acid was placed in a 15 milliliter polypropylene tube and 48 microliters of the polypeptide stock solution added to the tube and mixed (the "coating solution"). Wells of microwell plates were filled with 100ul of the coating solution of the polypeptides and 100ul/well of 0.24M carbonate/0.2N NaOH added to raise the coating solution to an alkaline pH. The plate was covered and allowed to stand overnight at room temperature. The coating solution was removed by aspiration and 300ul/well of Plate Blocking solution (containing per liter; 25g non-fat dry milk, 14.7g sodium citrate dihydrate, 8.47g sodium chloride and 0.05ml Antifoam A, 1.0 ml Kathon GC/ICP) was added and incubated for 1 hr. at room temperature. Blocking solution was removed by aspiration, and the plates were used immediately or allowed to air-dry and stored for later use. To carry out the immunoassay, plasma samples were diluted 20-fold in Specimen Diluent (containing per liter; 44.1g sodium citrate dihydrate, 1.2ml Tween 20, 50g non-fat dry milk, 0.05ml Antifoam A, 50ml goat serum, 58.6g 2-[N-morpholino]ethane sulfonic acid, 92.9g triethanolamine hydrochloride, 1ml Proclin 300) and 100ul was added to individual wells. Samples were incubated for 30 minutes at 37° C, then removed and the wells were washed five times with 0.1M NaCl/0.05% Tween 20 (350ul/wash). One hundred microliters of goat antihuman Ig-horseradish peroxidase conjugate diluted in citrate buffer, pH 7.0, containing 1% normal goat serum was added to each well for 30 minutes at 37° C prior to washing five times as above. The ELISA assay was developed by adding 100ul/well of substrate solution (80ug/ml tetramethylbenzidine, 0.0015% hydrogen peroxide in citrate/phosphate buffer, pH 6.0) for 30 minutes at room temperature. Reactions were stopped with the addition of 100ul of 1N H₂SO₄ per well, and the ratio of the optical density at 450 nm to 630 nm was determined by an automated ELISA reader. The cut-off value for a positive result was set at 0.200 Absorbance Units above the average absorbance obtained from at least three known negative samples.

The results in Table 1A shows the reactivity of the polypeptides of the invention with HIV-1 positive and negative samples wherein the HIV-1 positive samples are GS91-(034, 037, 042, 046, 049, 052, 056, 067), 11230, 11424, 11527, 11532 and 11535, and the negative samples are PS1059-PS1062, PS1068, PS1071, and D21-D25.

**TABLE 1A**

| Absorbance (450nm/630nm)** | | | | |
|---|---|---|---|---|
| Sample* | BRU124E | BRUI24FIX | BRU124F3X | BRU124EX*** |
| GS91-034 | **0.606** | **1.000** | **0.945** | n.d. |
| GS91-037 | **0.507** | **1.584** | **1.551** | n.d. |
| GS91-042 | **1.860** | **1.899** | **1.888** | n.d. |
| GS91-046 | **1.598** | **1.848** | **1.831** | n.d. |
| GS91-049 | **1.034** | **1.955** | **1.991** | n.d. |
| GS91-052 | **1.606** | **1.852** | **1.917** | n.d. |
| GS91-056 | **1.848** | **1.966** | **1.957** | n.d. |
| GS91-067 | **1.960** | **2.110** | **2.110** | n.d. |
| PS1071 | 0.036 | 0.040 | 0.046 | n.d. |
| PS1062 | 0.041 | 0.047 | 0.046 | n.d. |
| PS1070 | 0.034 | 0.039 | 0.043 | n.d. |
| PS1061 | 0.020 | 0.023 | 0.021 | n.d. |
| PS1069 | 0.034 | 0.039 | 0.039 | n.d. |
| PS1060 | 0.027 | 0.052 | 0.044 | n.d. |
| PS1068 | 0.028 | 0.039 | 0.037 | n.d. |
| PS1059 | 0.043 | 0.046 | 0.049 | n.d. |
| | | | | |
| 11535 | n.d. | n.d. | n.d. | **1.652** |
| 11527 | n.d. | n.d. | n.d. | **2.595** |
| 11532 | n.d. | n.d. | n.d | **2.912** |
| 11424 | n.d. | n.d. | n.d. | **2.676** |
| 11230 | n.d. | n.d. | n.d | **0.759** |
| D21 | n.d. | n.d. | n.d. | 0.096 |
| D22 | n.d. | n.d. | n.d. | 0.045 |
| D23 | n.d. | n.d. | n.d. | 0.053 |
| D24 | n.d. | n.d. | n.d. | 0.044 |
| D25 | n.d. | n.d. | n.d | 0.034 |

| | | | | |
|---|---|---|---|---|
| * Samples were diluted 1/40, instead of 1/20, in order to be able to compare the absorbance between different polypeptides. The GS91- (034, 037, 042, 046, 049, 052, 056, 067), 11230, 11424, 11527,11532, and 11535) are known HIV-1 positive samples; samples PS1059-PS1062, PS1068, PS1071, and D21-25 are known HIV-1 negative samples. ** Highlighted values are positive values based on the cut-off values established by 0.200 + average negative. Cut-Off = 0.238 (except BRU124EX) The Cut-Off value for BRU124EX = 0.254 ***The testing for the reactivity of BRU124EX was done separately at different date, using different samples. n.d. = not done (or tested) | | | | |

The results in Table 1B show the reactivity of the polypeptides of the invention with HIV-2 positive and negative samples wherein the HIV-1 positive samples are 92099, 92100, P-83, P-84 and P-86 and the negative samples are NBD1, NBD2, NBD3, AND NBD4.

**TABLE 1B**

| Absorbance (450nm/630nm)* | | | | | | | | |
|---|---|---|---|---|---|---|---|---|
| Sample | 41-2-3GC | E1 | EX | C1X | C2X | C3X | C5X | 2A1 |
| 92099 | **>3.00** | **0.41** | **0.73** | 0.24 | **1.61** | **0.78** | **1.77** | **1.91** |
| 92100 | **>3,00** | **0.47** | **0.75** | **0.26** | **1.91** | **0.88** | **1.82** | **1.86** |
| P-83 | **2.82** | **0.44** | **1.05** | 0.10 | **1.02** | **0.45** | **1.06** | 0.08 |
| P-84 | **>3.00** | **0.47** | **1.04** | **0.27** | **0.75** | **0.40** | **0.73** | 0.18 |
| P-86 | **>3.00** | 0.16 | 0.23 | 0.16 | **0.35** | 0.20 | **0.34** | 0.13 |
| NBD1 | 0.07 | 0.06 | 0.06 | 0.05 | 0.05 | 0.05 | 0.05 | 0.09 |
| NBD2 | 0.11 | 0.11 | 0.12 | 0.11 | 0.11 | 0.11 | 0.12 | 0.11 |
| NBD3 | 0.04 | 0.03 | 0.05 | 0.03 | 0.03 | 0.05 | 0.03 | 0.03 |
| NBD4 | 0.07 | 0.05 | 0.06 | 0.05 | 0.04 | 0.05 | 0.07 | 0.05 |

| | | | | | | | | |
|---|---|---|---|---|---|---|---|---|
| Highlighted values are positive values based on the cut-off values established by 0.200 + average Negative. Cut-Off = 0.256 Known positive samples are 92099, 92100, P-83, P-84 and P-86 and the known negative samples are NBD1, NBD2, NBD3, AND NBD4. | | | | | | | | |

An improvement on the specificity of HIV-1 and HIV-2 antibody detection by the incorporation of pol polypeptides (BRU124F3X and ROD124C5X) is illustrated by the study results shown in Table 1C. In this study, the pol polypeptides were coated on the microwell plate individually or together with envelope specific polypeptides, as mentioned in U.S. Patent No. 5,439,792, the teachings of which are hereby incorporated by reference. For the plate coating with individual pol polypeptides, the polypeptide was coated at 1.0 ug/ml. For the plate coating of the mixture of HIV-1 and HIV-2 polypeptides, the polypeptides were mixed together at the following concentrations in the coating buffer: 1.23 ug/ml for HIV-1 envelope polypeptide (designated as MNGC), 0.64 ug/ml for HIV-2 envelope polypeptide (designated as 41-2-3GC), 0.25 ug/ml for BRU124F3X and 0.125 ug/ml for ROD124C5X. The polypeptide coating procedure was the same as described earlier in the "immunoreactivity" section. The samples tested are known HIV-1 (Western blot all-band positive) samples (i.e., SAL040, SAL041, SAL059, SAL063, SAL064), known HIV-2 (Western blot all-band positive) samples (i.e., 52, GB92000128, GB92000152, GB92000154, GB92000158), HIV-1 indeterminate samples (i.e., B3113, B5813, B5885, B7045, C000127, C000214, C000455) and HIV-2 indeterminate samples (i.e., B3123, B5605, B5810, B5826, B5832, B5875, B6312). Also included are the control samples used in the Genetic Systems® HIV-1/HIV-2 Peptide EIA kit (available from Sanofi Diagnostics Pasteur, Inc., Redmond, Washington), namely HIV-1 positive control (PC-1), HIV-2 positive control (PC-2) and negative control (NC). Both the known positive and the indeterminate samples were also tested on a commercially available viral lysate based test, the Genetic Systems® HIV-1/HIV-2 EIA (Sanofi Diagnostics Pasteur, Inc., Redmond, Washington).

**TABLE 1C**

| **COMPARISON OF HIV-1/2 WESTERN BLOT ALL BAND POSITIVE AND INDETERMINATE SAMPLE REACTIVITIES IN ELISA WITH POL ONLY COATED PLATES AND FOUR PEPTIDE COATED PLATES** | | | | | | | | | | | | |
|---|---|---|---|---|---|---|---|---|---|---|---|---|
| | 4 Peptides* | | | HIV-1 pol(BRU124F3X) Only | | | HIV-2 pol (ROD124C5X) Only | | | | HIV-1/2 Viral Lysate*** | |
| Sample | A450/A630 | S/CO | Pos/Neg** | A450/A630 | S/CO | Pos/Neg** | A450/A630 | S/CO | Pos/Neg** | | S/CO | Pos/Neg** |
| PC-1 | **1.610** | **4.8** | **P** | **1.503** | **3.0** | P | 0.441 | 0.8 | N | | N.A. | N.A. |
| | **1.639** | **4.9** | **P** | **1.539** | **3.1** | P | 0.451 | 0.9 | N | | N.A. | N.A. |
| PC-2 | **1.259** | **3.8** | **P** | 0.307 | 0.6 | N | 0.438 | 0.8 | N | | N.A. | N.A. |
| | **1.221** | **3.7** | P | 0.301 | 0.6 | N | **0.560** | **1.1** | **P** | | N.A. | N.A. |
| NC | 0.093 | 0.3 | N | 0.254 | 0.5 | N | 0.279 | 0.5 | N | | N.A. | N.A. |
| | 0.091 | 0.3 | N | 0.265 | 0.5 | N | 0.302 | 0.6 | N | | N.A. | N.A. |
| Cut-off | 0.332 | N.A. | | 0.500 | N.A. | | 0.531 | N.A. | | | N.A. | N.A. |
| | | | | | | | | | | | | |
| HIV-1 WB Pos. | | | | | | | | | | | | |
| SAL040 | **2.985** | **9.0** | **P** | **2.956** | **5.9** | **P** | **2.709** | **5.1** | **P** | | **>8.0** | **P** |
| SAL041 | **3.028** | **9.1** | **P** | **2.876** | **5.8** | **P** | **2.555** | **4.8** | **P** | | **>8.0** | **P** |
| SAL059 | **2.957** | **8.9** | **P** | **2.796** | **5.6** | **P** | **2.238** | **4.2** | **P** | | **>8.0** | **P** |
| SAL063 | **3.009** | **9.1** | **P** | **2.790** | **5.6** | **P** | **2.714** | **5.1** | **P** | | **>8.0** | **P** |
| SAL064 | **2.934** | **8.8** | **P** | **2.893** | **5.8** | **P** | **2.865** | **5.4** | **P** | | **>8.0** | **P** |
| HIV-2 WB Pos. | | | | | | | | | | | | |
| 52 | **2.972** | **9.0** | **P** | **2.738** | **5.5** | **P** | **2.669** | **5.0** | **P** | | **>8.0** | **P** |
| GB92000128 | **2.939** | **8.9** | **P** | **2.698** | **5.4** | **P** | **2.872** | **5.4** | **P** | | **>8.0** | **P** |
| GB92000152 | **2.904** | **8.7** | **P** | **2.633** | **5.3** | **P** | **2.743** | **5.2** | **P** | | **>8.0** | **P** |
| GB92000154 | **3.011** | **9.1** | **P** | **2.677** | **5.4** | **P** | **2.857** | **5.4** | **P** | | **>8.0** | **P** |
| GB92000158 | **3.061** | **9.2** | **P** | **2.723** | **5.5** | **P** | **2.930** | **5.5** | **P** | | **>8.0** | **P** |
| HIV-1 | | | | | | | | | | | | |
| Indeterminates | | | | | | | | | | | | |
| B3113 | 0.094 | 0.3 | N | 0.346 | 0.7 | N | 0.253 | 0.5 | N | | **3.2** | **FP** |
| B5813 | 0.063 | 0.2 | N | 0.303 | 0.6 | N | 0.244 | 0.5 | N | | **3.0** | **FP** |
| B5885 | 0.063 | 0.2 | N | 0.152 | 0.3 | N | 0.154 | 0.3 | N | | **3.1** | **FP** |
| 87045 | 0.062 | 0.2 | N | 0.241 | 0.5 | N | 0.208 | 0.4 | N | | **3.5** | **FP** |
| C000127 | 0.050 | 0.2 | N | 0.172 | 0.3 | N | 0.177 | 0.3 | N | | **3.1** | **FP** |
| C000214 | 0.056 | 0.2 | N | 0.157 | 0.3 | N | 0.155 | 0.3 | N | | **5.4** | **FP** |
| C000455 | 0.065 | 0.2 | N | 0.136 | 0.3 | N | 0.179 | 0.3 | N | | **5.4** | **FP** |
| | | | | | | | | | | | | |
| HIV-2 | | | | | | | | | | | | |
| Indeterminates | | | | | | | | | | | | |
| B3123 | 0.051 | 0.2 | N | 0.142 | 0.3 | N | 0.188 | 0.4 | N | | **3.2** | **FP** |
| B5805 | 0.074 | 0.2 | N | 0.203 | 0.4 | N | 0.276 | 0.5 | N | | **3.8** | **FP** |
| B5810 | 0.053 | 0.2 | N | 0.204 | 0.4 | N | 0.205 | 0.4 | N | | **4.3** | **FP** |
| B5826 | 0.049 | 0.1 | N | 0.145 | 0.3 | N | 0.177 | 0.3 | N | | **3.0** | **FP** |
| B5832 | 0.057 | 0.2 | N | 0.183 | 0.4 | N | 0.195 | 0.4 | N | | **3.2** | **FP** |
| B5875 | 0.056 | 0.2 | N | 0.254 | 0.5 | N | 0.286 | 0.5 | N | | **3.8** | **FP** |
| B6312 | 0.027 | 0.1 | N | 0.150 | 0.3 | N | 0.155 | 0.3 | N | | **2.8** | **FP** |

| | | | | | | | | | | | | |
|---|---|---|---|---|---|---|---|---|---|---|---|---|
| * 4 peptides = MNGC (1.23ug/ml): 41-2-3GC (0.64ug/ml): BRU124F3X (0.25ug/ml): ROD124C5X (0.125ug/ml) HIV-1pol (BRU124F3X) Only and HIV-2 pol (124C5X) Only = 1.0ug/ml each HIV-1 and HIV-2 WB Pos. = All band Western blot Positive samples HIV-1 and HIV-2 Indeterminates = Western blot Indeterminates ** In Pos./Neg. columns : P = Positive; N = Negative; FP = False Positive *** Genetic Systems® HIV-1/2 EIA (FDA approved) | | | | | | | | | | | | |

Table 1C shows that all of the known HIV-1 and HIV-2 positive samples showed positive results when using either a single pol polypeptide coated plate or the plate coated with all four polypeptides. All HIV-1 and HIV-2 indeterminate samples showed negative results when using either a single pol polypeptide coated plate or the plate coated with all four polypeptides. The indeterminate samples showed highly positive results (false positive) when tested using the viral lysate-based HIV-1/HIV-2 EIA. These results very clearly show that the polypeptide based EIA incorporating the pol polypeptides of the invention is highly sensitive and specific in detecting HIV positive samples.

### Example 3 - Cloning pol23 and pol7:

Both *pol*23 and *pol*7 recombinants were initially cloned in the pGEX system (Phamiacia). The cloned inserts were then moved into other expression vectors that provide "purification handles" to simplify analysis of gene expression.

### Recombinant: pGEX/pol23

A PCR product was generated using HIV-1 viral DNA isolated from HIV-1 LAI strain viral lysate as a template, and the primer pair 5' AGCACCATGGGGATCCCAGGGATTAGATATCAGTACAATG 3' (SEQ ID NO. 16) and 5' AGTCAGAATTCATTGGCCTTGCCCCTGCTT 3' (SEQ ID NO. 17). PCR reactions used *UlT*ma DNA polymerase (Perkin-Elmer) to minimize the possibility of mutation due to polymerase error. The PCR product was digested with the restriction enzymes *Bam*HI and *Eco*RI and inserted into *Bam*HI, *Eco*RI-digested pGEX 5X-1 (Pharmacia). The ligation mix was transformed into *E. coli* DH11S and ampicillin-resistant colonies selected (see Maniatis, *supra*).

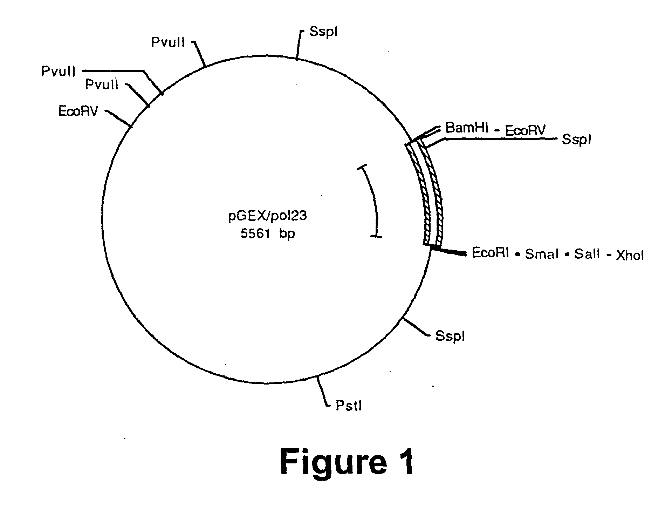

Insert-containing colonies were initially identified by colony-PCR. This was followed by growth, plasmid isolation¹, and restriction analysis to demonstrate that the candidate clones contained the predicted restriction sites (see Maniatis, *supra*). A plasmid map of the resulting clone is provided in Figure 1.
¹ Plasmid DNA was prepared from recombinant bacterial cultures grown overnight at 37°C in L-broth plus 100mg/ml ampicillin using the QIAwell 8 Plus Plasmid Kit (QIAGEN).

### Recombinant: pGEX/pol7.0

As described above, a PCR product was generated using a HIV-1 viral DNA template, and the primer pair 5' AGCACCATGGGGATCCCCTACAGTGCAGGGGAAAGAATA 3' (SEQ ID NO. 18) and 5' GACTAGTCGACTCAATCATCACCTGCCATCTG 3' (SEQ ID NO. 19). PCR reactions used *UlT*ma DNA polymerase (Perkin-Elmer) to minimize the possibility of mutation due to polymerase error. The PCR product was digested with the restriction enzymes *Bam*HI and *Sal*I and inserted into *Bam*HI, *Sal*I I-digested pGEX 5X-1. The ligation mix was transformed into *E. coli* DH11S and ampicillin-resistant colonies selected.

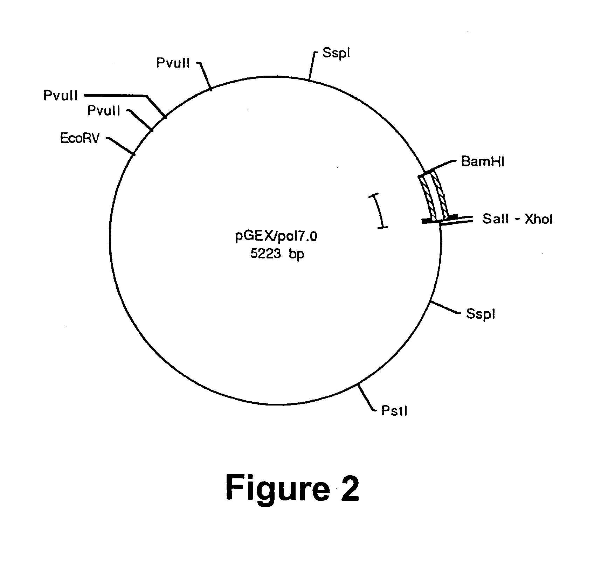

Insert-containing colonies were initially identified by colony-PCR. This was followed by growth, plasmid isolation, and restriction analysis to demonstrate that the candidate clones contained the predicted restriction sites. A plasmid map of the resulting clone is provided in Figure 2.

### Subcloning of pGEX/pol23 and pGEX/pol7.0 into expression vectors pOE42 and pThioHisA

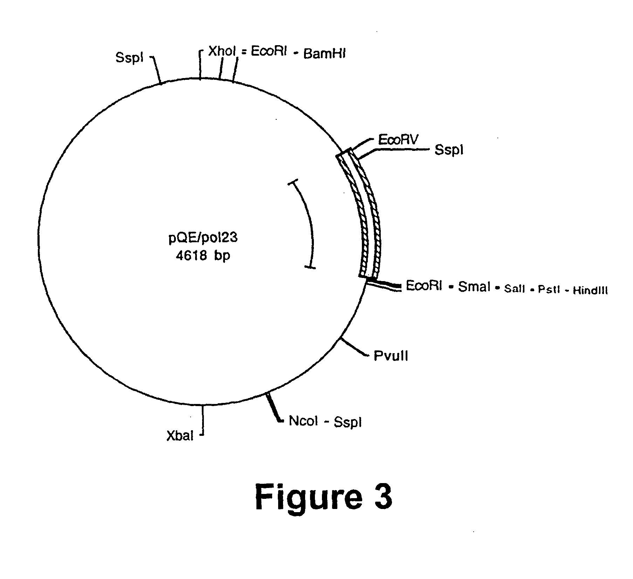

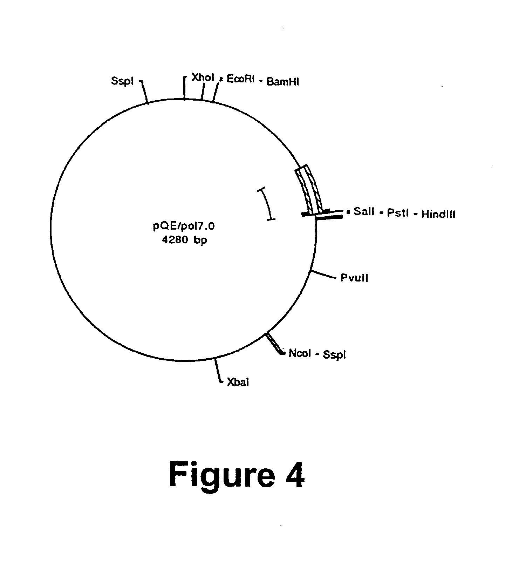

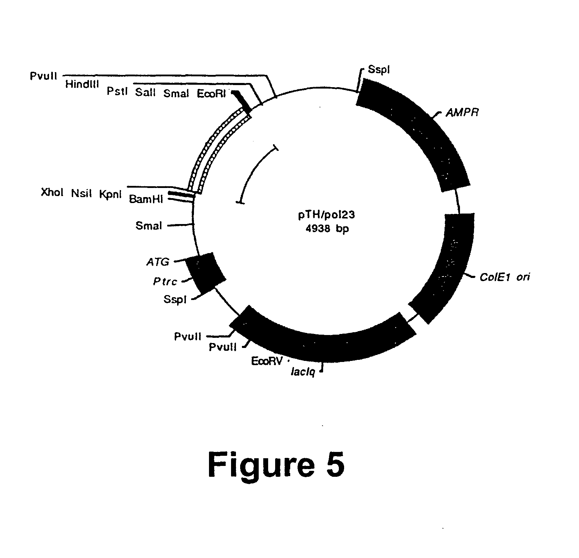

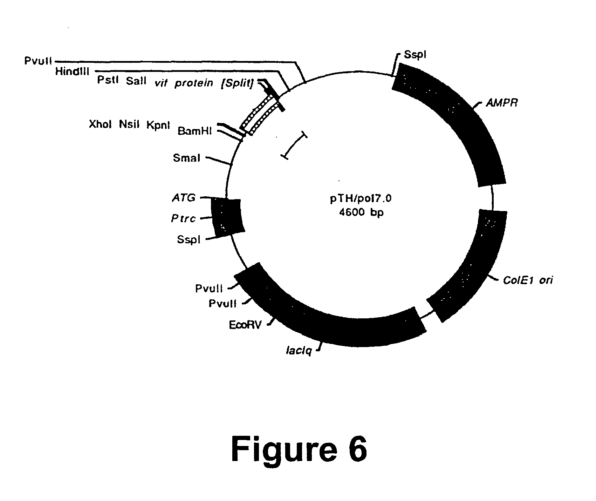

Insert DNA was prepared from pGEX/*pol*23 and pGEX/*pol*7.0 by digestion with the enzymes *Bam*HI and *Sal*I. The commercial expression vectors pThioHisA (Invitrogen) and pQE42 (QIAGEN) were each digested with the enzymes *Bgl*II² and *Sal*I. In separate ligation reactions, each insert was joined to each vector. (see Maniatis, *supra*) Again, ampicillin-resistant transformants were selected in the *E. coli* host strain DH11S. Identity of the clones was verified by colony-PCR and restriction analysis of purified plasmid DNA. The resulting recombinants were designated pQE/*pol*23, pQE/*pol*7.0, pThioHis/*pol*23, and pThioHis/*pol*7.0. (Plasmid maps of the resulting clones are provided in Figures 3 through 6 respectively).
² Note that the enzymes *Bam*HI and *Bgl*II produce cohesive ends that can be joined by ligation.

### Example 4 - Protein Expression of Example 3 Clones:

All three plasmid expression systems, pGEX, pThioHisA, and pQE42, employing the tac promoter are negatively regulated (shut off) by glucose, and positively regulated (turned on) by the lactose analog IPTG.

Twenty-five ml cultures of each of the recombinants pQE/*pol*23, pQE/*pol*7.0, pThioHis/*pol*23, and pThioHis/*pol*7.0 were grown in complete T-broth plus 100mg/ml ampicillin, 1.0% glucose at 37°C (with shaking) until the optical density at 600 nm was between 2 and 6. Cells were then pelleted and resuspended in fresh glucose-free medium that contained 1mM IPTG to induce expression of the recombinant fusion protein. After 2 additional hours of incubation, the induced cultures were harvested by centrifugation at 2000 rpm for 10 minutes. The resulting supernatant medium was discarded and the cell pellets frozen at -70°C.

Frozen pellets were thawed and resuspended in 2.0 ml of an aqueous medium of 20 mM sodium phosphate, pH 7.8 and 500 mM NaCl. The cells were lysed by two cycles of freezing in a dry ice/ethanol bath followed by thawing in warm water and 10 short bursts of sonication. The resulting lysate was then centrifuged in an Eppendorf microfuge at maximum speed until pelleted. The lysate supernatants and pellets were used for further purification.

### pQE/pol23, pQE/pol7.0: Ni-NTA purification

For these recombinants, the lysate pellets were dissolved in an aqueous buffer comprising 20 mM sodium phosphate, pH 7.8 and 8M urea. This suspension was bound to Ni-NTA agarose (QIAGEN) that was pre-equilibrated in the same buffer. Unbound material was washed away using the same buffer. Bound protein was then eluted with an aqueous buffer comprising 20 mM sodium phosphate, 6.4 M urea and 100 mM EDTA. The resulting purified fusion proteins were analyzed by SDS-PAGE.

The lysate supernatants were also purified, using methods found below, however higher levels of recombinant protein were found in the lysate pellets.

### pThioHis/pol23, pThioHis/pol7.0: Ni-NTA purification

For these recombinants, lysate supernatants were bound to Ni-NTA that was pre-equilibrated in an aqueous buffer comprising 20 mM sodium phosphate, pH 7.8 and 500 mM NaCl. Unbound material was washed away with the same buffer, and then again with an aqueous wash buffer (20 mM sodium phosphate, pH 6.0, 500 mM NaCl).

Bound protein was eluted in four successive steps using increasing concentration of imidazole in the wash buffer (50 mM, 200 mM, 350 mM, 500 mM). SDS-PAGE analysis showed that 350 mM imidazole was the optimal elution condition for these recombinants.

### SDS-PAGE and Western Blot Analysis

Using standard laboratory procedures, samples of the resulting partially purified fusion proteins were separated by SDS-Page. Novex 4-20% polyacrylamide gradient gels were used.

In all cases it was observed that the recombinant fusion proteins migrated close to the predicted sizes as found in Table 2.

**TABLE 2**

| **Recombinant** | **Predicted fusion protein molecular weight** | **Purified from** |
|---|---|---|
| pQE/po123 | 46 kD | lysate pellet |
| pQE/po17.0 | 33.5 kD | lysate pellet |
| pThioHis/po123 | 37 kD | lysate supernatant |
| pThioHis/po17.0 | 24 kD | lysate supernatant |

The separated proteins were also transferred to a nitrocellulose support for Western blot analysis. The semi-dry electrophoretic transfer method (Harlow and Lane, Antibodies, a laboratory manual pp. 488-489) was used to transfer electrophoresed proteins to nitrocellulose membranes.

After transfer, membranes were blocked with Blotto (50 mM Tris pH7.5, 150 mM NaCl, 5.0% nonfat dry milk, 0.1 % Tween-20) either overnight at 4°C or one hour at room temperature. After removing the blocking agent, the membrane was incubated with the primary antibody³ diluted in Blotto, for one hour at room temperature. The primary antibody solution is then discarded and the blot is washed four times for 5 minutes each with Blotto. The secondary antibody-enzyme conjugate⁴ is then added for a 30 minute room temperature incubation. After 30 minutes, the secondary antibody is discarded and the blot is again washed four times as earlier. This is followed by a rinse with water and then addition of the color substrate solution⁵.
³ Two mouse monoclonal antibodies against HIV *pol* were used at 1:4000 dilutions. A human serum positive to HIV was used at a 1:500 dilution.
⁴ The secondary antibody conjugate was either horseradish peroxidase-conjugated goat-antimouse antibody (1:2000) in Blotto if the primary antibody was mouse monoclonal or alkaline phosphatase-conjugated goat-antihuman antibody (1:2000) in Blotto if the primary antibody was human serum.
⁵ Tetramethylbenzidine (TMB) (Vector Labs) was the substrate used with the horseradish peroxidase secondary antibody conjugate, while BCIP/NBT One-Component Substrate (Kirkegaard & Perry Laboratories Inc.) was used with the alkaline phosphatase secondary antibody conjugate.

Color development was stopped by washing with water after bands appeared. The pQE/*pol*23 and pThioHis/*pol*23 protein bands both reacted in Western blots using one of the mouse monoclonal antibodies, while the pQE/*pol*7.0 and pThioHis/*pol*7.0 protein bands both reacted with the other. All four band were detected with the human HIV-1 positive serum.

### Example 5 - Enzyme Immunoassay (EIA) Testing:

EIA plates were coated with the partially purified recombinant fusion proteins. Each sample was coated at a dilution of 1:500 in 15 mMcarbonate/35 mM bicarbonate coating buffer pH 9.6 overnight at room temperature. The coated plates were then "ON/OFF" blocked (2.5% non-fat dry milk), "ON/OFF" coated with a 4% sucrose-solution, and air-dried overnight. As prepared, the plates may be stored in a sealed pouch with desiccant.

Each recombinant fusion protein was tested for reactivity with 16 HIV-1 positive samples, 8 normal donor samples, and 4 *E. coli*-reactive samples. The *E. coli*-reactive samples are samples that have previously been shown to have high reactivity to proteins from *E. coli* lysate. These were included to ensure that the reactivity we observe with HIV-1-positive samples is specific to the recombinant polypeptide and not directed toward contaminating *E. coli* proteins.

Briefly, the samples were diluted 1:101 in a specimen diluent (containing per liter; 44.1g sodium citrate dihydrate, 1.2ml Tween 20, 50g non-fat dry milk, 0.05ml Antifoam A, 50ml goat serum, 58.6g 2-[N-morpholino]ethane sulfonic acid, 92.9g triethanolamine hydrochloride, 1ml Proclin 300) and 200 µl of the resulting mixtures were added to each well of the previously prepared plates. The plates were covered and incubated at 37 ± 1° C for 60 minutes. The fluid was aspirated from each well and the plate was washed a minimum of 5 times with a wash solution (0.1M NaCl/0.05% Tween 20).

100 µl of a conjugate solution (goat antihuman Ig-horseradish peroxidase conjugate diluted in citrate buffer, pH 7.0, containing 1% normal goat serum) was added to each well. The plates were then covered and incubated for 60 minutes at 37 ± 1° C. Following incubation, the fluid was aspirated from each well and the plate was washed a minimum of 5 times with the wash solution.

100 µl of a chromogen solution (80ug/ml tetramethylbenzidine, 0.0015% hydrogen peroxide in citrate/phosphate buffer, pH 6.0) was added to each well. The plates were then covered and incubated in the dark for 30 minutes at room temperature. After removing the cover and adding 100 µl of a stopping reagent (1N H₂SO₄) the plates were read at an absorbance of 450 nm with 615 nm to 630 nm as a reference. The ratio of the optical densities at 450 nm to 630 nm was calculated. The cut-off value for a positive result was set at 0.200 Absorbance Unites above the average absorbance obtained from at least three known negative samples. The results of these procedures are presented in Table 3.

**TABLE 3**

| | **Coated Plates** | | | |
|---|---|---|---|---|
| ***Samples*** | ***pQE*/*pol23*** | ***pQE*/*pol7.0*** | ***pTH*/*pol23*** | ***pTH*/*po17.0*** |
| ***B7095*** | 2.122 | 1.903 | 0.557 | 1.68 |
| ***C000218*** | 1.553 | 2.11 | 0.046 | 2.042 |
| ***C000582*** | 1.557 | 1.618 | 0.208 | 1.219 |
| ***B3137*** | 2.075 | 2.155 | 0.949 | 1.933 |
| ***B5878*** | 2.142 | 0.107 | 0.732 | 0.064 |
| ***B5879*** | 2.255 | 2.13 | 1.075 | 2.14 |
| ***B5847*** | 0.042 | 0.132 | 0.01 | 0.049 |
| ***C000024*** | 0.051 | 2.278 | 0.018 | 2.225 |
| ***B5853*** | 1.671 | 2.212 | 0.509 | 2.154 |
| ***B3138*** | 0.916 | 1.732 | 0.2 | 1.295 |
| ***B5895*** | 0.944 | 0.717 | 0.027 | 0.587 |
| ***C000130*** | 0.438 | 0.598 | 0.021 | 0.299 |
| ***C000136*** | 0.947 | 1.622 | 0.08 | 1.385 |
| ***B5889*** | 1.438 | 1.139 | 0.25 | 0.649 |
| ***B5822*** | 0.331 | 0.05 | 0.051 | 0.031 |
| ***SAL054*** | 2.044 | 1.802 | 0.955 | 1.771 |
| | | | | |
| ***PSBC1171*** | 0.07 | 0.021 | 0.019 | 0.014 |
| ***PSBC1265*** | 0.033 | 0.019 | 0.014 | 0.018 |
| ***PSBC1341*** | 0.014 | 0.01 | 0.009 | 0.01 |
| ***PSBC1398*** | 0.028 | 0.015 | 0.013 | 0.013 |
| ***PSBC1481*** | 0.027 | 0.017 | 0.014 | 0.016 |
| ***PSBC1521*** | 0.021 | 0.023 | 0.013 | 0.014 |
| ***PSBC1657*** | 0.028 | 0.017 | 0.014 | 0.011 |
| ***PSBC1566*** | 0.017 | 0.012 | 0.011 | 0.01 |
| | | | | |
| ***EC+1*** | 0.039 | 0.028 | 0.015 | 0.025 |
| ***EC+2*** | 0.046 | 0.074 | 0.021 | 0.03 |
| ***EC+3*** | 0.048 | 0.024 | 0.014 | 0.03 |
| ***EC+4*** | 0.022 | 0.015 | 0.011 | 0.023 |

It is evident from the foregoing results that by employing one or a combination of polypeptides of the subject invention, a sensitive, accurate test for the presence of antibodies to HIV is provided. The subject polypeptides can be used by themselves or in combination with a screening assay or confirmatory assay, whereas the complete lysate or complete antigens may be employed as an independent procedure. The subject polypeptides can also be combined with polypeptides or proteins derived from the envelope or gag regions of HIV-1 or HIV-2 in a screening assay or confirmatory assay. Furthermore, because of the specificities of the polypeptides, one could anticipate that the DNA sequences coding for the polypeptides would also find similar specificity in a DNA hybridization assay.

## Claims

1. A method for determining the presence of antibodies to HIV in a body fluid, comprising:
(a) contacting under conditions which permit immunospecific binding to form a reaction mixture the body fluid with a composition containing at least one polypeptide or protein consisting of the amino acid sequences: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys- or one or more amino acids added to facilitate coupling; and Z is OH or NH;
(b) detecting whether immunospecific binding has occurred between the polypeptide and an antibody component of the body fluid in which an immune complex is formed and in which the detection of the immune complex indicates the presence of antibodies to HIV in the body fluid.

2. The method according to claim 1 in which the polypeptide is conjugated to a carrier macromolecule.

3. The method according to claim 1 in which the polypeptide is immobilized.

4. The method according to claim 1 in which the immunospecific binding is detected by immunoprecipitation.

5. The method according to claim 1 in which the composition includes at least one polypeptide selected from a polymerase protein of HIV-1 and one selected from a polymerase protein of HIV-2.

6. The method according to claim 1 in which immunospecific binding between the polypeptide or protein and the antibody component of the body fluid is detected by:
(i) removing unbound component from immune complexes formed in the immunoreaction mixture;
(ii) adding a labeled antibody to the immunoreaction mixture, the labeled antibody being capable of immunospecifically binding to a component of the immune complexes and the label providing a detectable signal; and
(iii) determining whether the labeled antibody binds to the immune complexs.

7. The method according to claim 6 in which the label comprises an enzyme which is detected by the addition of the enzyme substrate.

8. The method according to claim 6 in which the label comprises a radiolabel.

9. The method according to claim 6 in which the label comprises a fluorescent label.

10. A method for determining the presence of antibodies to HIV-1 in a body fluid, comprising:
(a) contacting under conditions which permit immunospecific binding to form a reaction mixture the body fluid with a composition containing at least one polypeptide or protein consisting of the following amino acid sequences: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys- or one or more amino acids added to facilitate coupling; and Z is OH or NH;
(b) determining whether immunospecific binding has occurred between the polypeptide and an antibody component of the body fluid in which the detection of the immune complex indicates the presence of antibodies to HIV in the body fluid.

11. A polypeptide composition, immunoreactive to antibodies to HIV, consisting of one of the amino acid sequences: wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys- or one or more amino acids added to facilitate coupling; and Z is OH or NH₂.

12. The polypeptide composition of claim 11, wherein said polypeptide has formula (III) BRU124F1X.

13. The polypeptide composition of claim 11, wherein said polypeptide has formula (IV) BRU124F3X.

14. The polypeptide composition of claim 11, wherein said polypeptide has formula (XIII) *pol*7-aa.

15. A polypeptide composition, immunoreactive with HIV-1, comprising a polypeptide encoded by a polynucleotide sequence, wherein said polynucleotide sequence is:

16. A test kit for determining the presence of antibodies to HIV in a body fluid, comprising an antigen and ancillary reagents suitable for use in detecting the presence of antibodies to the antigen in said fluid; wherein said antigen is the polypeptide of claim 11.

17. The test kit of claim 16, wherein said antigen is the polypeptide of claim 15.

## Patentansprüche

1. Verfahren zum Bestimmen der Anwesenheit von Antikörpern gegen HIV in einer Körperflüssigkeit, umfassend:
(a) in Kontakt bringen der Körperflüssigkeit unter Bedingungen die eine immunspezifische Bindung erlauben um ein Reaktionsgemisch zu bilden, mit einer Zusammensetzung enthaltend mindestens ein Polypeptid oder Protein bestehend aus den Aminosäuresequenzen: wobei
W entweder ein H der aminoterminalen NH₂ Gruppe des Polypeptids oder eine oder mehrere zusätzliche Aminosäuren ist, die an die aminoterminale NH₂ Gruppe des Polypeptids gebunden sind, wobei die zusätzlichen Aminosäuren so ausgewählt sind, um die Kopplung des Polypeptids an ein Trägerprotein oder einen Träger zu erleichtern; X fehlt, Cys-Gly-Gly oder Lys-Lys ist; Y fehlt, Cys- oder eine oder mehrere Aminosäuren ist, die hinzugefügt wurden, um die Kopplung zu erleichtern; und Z OH oder NH ist;
(b) Nachweisen, ob eine immunspezifische Bindung zwischen dem Polypeptid und einer Antikörperkomponente der Körperflüssigkeit aufgetreten ist, wodurch ein Immunkomplex gebildet wird, und wobei der Nachweis des Immunkomplex die Anwesenheit von Antikörpern gegen HIV in der Körperflüssigkeit anzeigt.

2. Verfahren nach Anspruch 1, wobei das Polypeptid an ein Trägermakromolekül konjugiert ist.

3. Verfahren nach Anspruch 1, wobei das Polypeptid immobilisiert ist.

4. Verfahren nach Anspruch 1, wobei die immunspezifische Bindung durch Imnmunopräzipitation nachgewiesen wird.

5. Verfahren nach Anspruch 1, wobei die Zusammensetzung mindestens ein Polypeptid ausgewählt aus einem Polymerase-Protein von HIV-1 und einem ausgewählt aus einem Polymerase-Protein von HIV-2 enthält.

6. Verfahren nach Anspruch 1, wobei die immunspezifische Bindung zwischen dem Polypeptid oder Protein und der Antikörperkomponente der Körperflüssigkeit nachgewiesen wird durch:
(i) Entfernen von nicht gebundener Komponente von den in dem Immunreaktionsgemisch gebildeten Immunkomplexen;
(ii) Hinzufügen eines markierten Antikörpers zu dem Immunreaktionsgemisch, wobei der markierten Antikörper in der Lage ist, immunspezifisch an eine Komponente der Immunkomplexe zu binden und der Marker ein nachweisbares Signal zur Verfügung stellt; und
(iii) Nachweisen, ob der markierte Antikörper an den Immunkomplex bindet.

7. Verfahren nach Anspruch 6, wobei der Marker ein Enzym umfaßt, das durch die Hinzufügung des Enzymsubstrats nachgewiesen wird.

8. Verfahren nach Anspruch 6, wobei der Marker eine Radiomarkierung umfaßt.

9. Verfahren nach Anspruch 6, wobei der Marker eine fluoreszente Markierung umfaßt.

10. Verfahren zum Bestimmen der Anwesenheit von Antikörpern gegen HIV-1 in einer Körperflüssigkeit, umfassend:
(a) in Kontakt bringen der Körperflüssigkeit unter Bedingungen die eine immunspezifische Bindung erlauben um ein Reaktionsgemisch zu bilden, mit einer Zusammensetzung enthaltend mindestens ein Polypeptid oder Protein bestehend aus den folgenden Aminosäuresequenzen: wobei
W entweder ein H der aminoterminalen NH₂ Gruppe des Polypeptids oder eine oder mehrere zusätzliche Aminosäuren ist, die an die aminoterminale NH₂ Gruppe des Polypeptids gebunden sind, wobei die zusätzlichen Aminosäuren so ausgewählt sind, um die Kopplung des Polypeptids an ein Trägerprotein oder einen Träger zu erleichtern; X fehlt, Cys-Gly-Gly oder Lys-Lys ist; Y fehlt, Cys- oder eine oder mehrere Aminosäuren ist, die hinzugefügt wurden, um die Kopplung zu erleichtern; und Z OH oder NH ist;
(b) Nachweisen, ob eine immunspezifische Bindung zwischen dem Polypeptid und einer Antikörperkomponente der Körperflüssigkeit aufgetreten ist, wobei der Nachweis des Immunkomplex die Anwesenheit von Antikörpern gegen HIV in der Körperflüssigkeit anzeigt.

11. Polypeptidzusammensetzung, die immunreaktiv auf Antikörper gegen HIV ist, bestehend aus einer der Aminosäuresequenzen: wobei
W entweder ein H der aminoterminalen NH₂ Gruppe des Polypeptids oder eine oder mehrere zusätzliche Aminosäuren ist, die an die aminoterminale NH₂ Gruppe des Polypeptids gebunden sind, wobei die zusätzlichen Aminosäuren so ausgewählt sind, um die Kopplung des Polypeptids an ein Trägerprotein oder einen Träger zu erleichtern; X fehlt, Cys-Gly-Gly oder Lys-Lys ist; Y fehlt, Cys- oder eine oder mehrere Aminosäuren ist, die hinzugefügt wurden, um die Kopplung zu erleichtern; und Z OH oder NH ist.

12. Polypeptidzusammensetzung nach Anspruch 11, wobei das Polypeptid die Formel (III) BRU124F1X aufweist.

13. Polypeptidzusammensetzung nach Anspruch 11, wobei das Polypeptid die Formel (IV) BRU124F3X aufweist.

14. Polypeptidzusammensetzung nach Anspruch 11, wobei das Polypeptid die Formel (XIII) *pol*7-aa aufweist.

15. Polypeptidzusammensetzung, die immunreaktiv mit HIV-1 ist, umfassend ein Polypeptid, das durch eine Polynukleotidsequenz kodiert wird, wobei die Polynukleotidsequenz ist:

16. Testkit zum Nachweisen der Anwesenheit von Antikörpern gegen HIV in einer Körperflüssigkeit, umfassend ein Antigen und Hilfsreagenzien, die zur Verwendung beim Nachweis der Anwesenheit von Antikörpern gegen das Antigen in der Flüssigkeit geeignet sind; wobei das Antigen das Polypeptid von Anspruch 11 ist.

17. Testkit nach Anspruch 16, wobei das Antigen das Polypeptid von Anspruch 15 ist.

## Revendications

1. Procédé destiné à déterminer la présence d'anticorps anti-VIH dans un fluide corporel comprenant :
(a) la mise en contact du fluide corporel, dans des conditions qui permettent une liaison immunospécifique pour former un mélange réactionnel, avec une composition contenant au moins un polypeptide ou une protéine constitué(e) des séquences d'acides aminés : dans lesquelles W représente, soit un atome H du groupe NH₂ amino-terminal du polypeptide, soit un ou plusieurs acides aminés supplémentaires liés au groupe NH₂ amino-terminal du polypeptide, les acides aminés supplémentaires étant sélectionnés pour faciliter un couplage du polypeptide à une protéine porteuse ou à un support ; X est absent, Cys-Gly-Gly ; ou Lys-Lys, Y est absent, Cys- ou un ou plusieurs acides aminés ajoutés pour faciliter le couplage ; et Z représente OH ou NH ;
(b) la détection quant à savoir si une liaison immunospécifique est apparue entre le polypeptide et un composant anticorps du fluide corporel dans lequel un complexe immun est formé et dans lequel la détection du complexe immun indique la présence d'anticorps anti-VIH dans le fluide corporel.

2. Procédé selon la revendication 1, dans lequel le polypeptide est conjugué à une macromolécule porteuse.

3. Procédé selon la revendication 1, dans lequel le polypeptide est immobilisé.

4. Procédé selon la revendication 1, dans lequel la liaison immunospécifique est détectée par immuno-précipitation.

5. Procédé selon la revendication 1, dans lequel la composition inclut au moins un polypeptide choisi à partir d'une protéine polymérase de VIH-1 et un polypeptide choisi à partir d'une protéine polymérase de VIH-2.

6. Procédé selon la revendication 1, dans lequel une liaison immunospécifique entre le polypeptide ou la protéine et le composant anticorps du fluide corporel est détectée par :
(i) élimination du composant non lié des complexes immuns formés dans le mélange immunoréactionnel ;
(ii) addition d'un anticorps marqué au mélange immunoréactionnel, l'anticorps marqué étant capable de se lier immunospécifiquement à un composant des complexes immuns et le marqueur fournissant un signal détectable ; et
(iii) détermination quant à savoir si l'anticorps marqué se lie aux complexes immuns.

7. Procédé selon la revendication 6, dans lequel le marqueur comprend une enzyme qui est détectée on ajoutant le substrat enzymatique.

8. Procédé selon la revendication 6, dans lequel le marqueur comprend un radiomarqueur.

9. Procédé selon la revendication 6, dans lequel le marqueur comprend un marqueur fluorescent.

10. Procédé destiné à déterminer la présence d'anticorps anti-VIH-1 dans un fluide corporel, comprenant :
(a) la mise en contact du fluide corporel, dans des conditions qui permettent une liaison immunospécifique pour former un mélange réactionnel, avec une composition contenant au moins un polypeptide ou une protéine constitué(e) des séquences d'acides aminés suivantes : dans lesquelles W représente, soit un atome H du groupe NH₂ amino-terminal du polypeptide, soit un ou plusieurs acides aminés supplémentaires liés au groupe NH₂ amino-terminal du polypeptide, les acides aminés supplémentaires étant sélectionnés pour faciliter un couplage du polypeptide à une protéine porteuse ou à un support ; X est absent, Cys-Cly-Cly ; ou Lys-Lys, Y est absent, Cys- ou un ou plusieurs acides aminés ajoutés pour faciliter le couplage ; et Z représente OH ou NH ;
(b) la détection quant à savoir si une liaison immunospécifique est apparue entre le polypeptide et un composant anticorps du fluide corporel dans lequel la détection du complexe immun indique la présence d'anticorps anti-VIH dans le fluide corporel.

11. Composition de polypeptide, immunoréactive envers des anticorps anti-VIH, constituée de l'une des séquences d'acides aminés : dans lesquelles W représente, soit un atome H du groupe NH₂ amino-terminal du polypeptide, soit un ou plusieurs acides aminés supplémentaires liés au groupe NH₂ amino-terminal du polypeptide, les acides aminés supplémentaires étant sélectionnés pour faciliter un couplage du polypeptide à une protéine porteuse ou à un support ; X est absent, Cys-Gly-Gly ; ou Lys-Lys, Y est absent, Cys- ou un ou plusieurs acides aminés ajoutés pour faciliter le couplage ; et Z représente OH ou NH₂.

12. Composition de polypeptide selon la revendication 11, dans laquelle ledit polypeptide possède la formule (III) BRU124F1X.

13. Composition de polypeptide selon la revendication 11, dans laquelle ledit polypeptide possède la formule (IV) BRU124F3X.

14. Composition de polypeptide selon la revendication 11, dans laquelle ledit polypeptide possède la formule (XIII) *pol*7-aa.

15. Composition de polypeptide, immunoréactive avec le VIH-1, comprenant un polypeptide codé par une séquence polynucléotidique, dans laquelle ladite séquence polynucléotidique est :

16. Kit de test destiné à déterminer la présence d'anticorps anti-VIH dans un fluide corporel, comprenant un antigène et des réactifs ancillaires adéquats pour utilisation pour détecter la présence d'anticorps dirigés contre l'antigène dans ledit fluide ; ledit antigène étant le polypeptide selon la revendication 11.

17. Kit de test selon la revendication 16, dans lequel ledit antigène est le polypeptide selon la revendication 15.


### SEQUENCE LISTING

<110> Genetic Systems Corporation
<120> SYNTHETIC ANTIGEN FOR THE DETECTION OF ANTIBODIES IMMUNOREACTIVE WITH HIV VIRUS
<130> 9197-88PC
<140> PCT/US98/16160
   <141> 1998-07-31
<150> 08/904,826
   <151> 1997-08-01
<160> 19
<170> PatentIn Ver. 2.1
<210> 1
   <211> 30
   <212> PRT
   <213> Human immunodeficiency virus type 1
<400> 1
   Lys Ile Gln Asn Phe Arg Val Tyr Tyr Arg Asp Ser Arg Asp Pro Leu
    1               5                  10                  15
   Trp Lys Gly Pro Ala Lys Leu Leu Trp Lys Gly Glu Gly Ala
               20                  25                  30
<210> 2
   <211> 36
   <212> PRT
   <213> Human immunodeficiency virus type 1
<400> 2
   Leu Gln Lys Gln Ile Thr Lys Ile Gln Asn Phe Arg Val Tyr Tyr Arg
    1               5                  10                  15
   Asp Ser Arg Asp Pro Leu Trp Lys Gly Pro Ala Lys Leu Leu Trp Lys
               20                  25                  30
   Gly Glu Gly Ala
           35
<210> 3
   <211> 40
   <212> PRT
   <213> Human immunodeficiency virus type 1
<400> 3
   Lys Ile Gln Asn Phe Arg Val Tyr Tyr Arg Asp Ser Arg Asp Pro Leu
    1               5                  10                  15
   Trp Lys Gly Pro Ala Lys Leu Leu Trp Lys Gly Glu Gly Ala Val Val
               20                  25                  30
   Ile Gln Asp Asn Ser Asp Ile Lys
           35                  40
<210> 4
   <211> 36
   <212> PRT
   <213> Human immunodeficiency virus type 1
<400> 4
   Lys Ile Gln Asn Phe Arg Val Tyr Tyr Arg Asp Ser Arg Asp Pro Leu
   1 5 10 15
   Trp Lys Gly Pro Ala Lys Leu Leu Trp Lys Gly Glu Gly Ala Val Val
   20 25 30
   Ile Gln Asp Asn
   35
<210> 5
   <211> 30
   <212> PRT
   <213> Human immunodeficiency virus type 2
<400> 5
   Lys Leu Lys Asp Phe Arg Val Tyr Phe Arg Glu Gly Arg Asp Gln Leu
   1 5 10 15
   Trp Lys Gly Pro Gly Glu Leu Leu Trp Lys Gly Glu Gly Ala
   20 25 30
<210> 6
   <211> 30
   <212> PRT
   <213> Human immunodeficiency virus type 2
<400> 6
   Lys Ile Gln Asn Phe Arg Val Tyr Tyr Arg Asp Ser Arg Asp Pro Leu
   1 5 10 15
   Trp Lys Gly Pro Ala Lys Leu Leu Trp Lys Gly Glu Gly Ala
   20 25 30
<210> 7
   <211> 40
   <212> PRT
   <213> Human immunodeficiency virus type 2
<400> 7
   Lys Leu Lys Asp Phe Arg Val Tyr Phe Arg Glu Gly Arg Asp Gln Leu
   1 5 10 15
   Trp Lys Gly Pro Gly Glu Leu Leu Trp Lys Gly Glu Gly Ala Val Leu
   20 25 30
   Val Lys Val Gly Thr Asp Ile Lys
   35 40
<210> 8
   <211> 33
   <212> PRT
   <213> Human immunodeficiency virus type 2
<400> 8
   Tyr Phe Arg Glu Gly Arg Asp Gln Leu Trp Lys Gly Pro Gly Glu Leu
   1               5                   10                  15
   Leu Trp Lys Gly Glu Gly Ala Val Leu Val Lys Val Gly Thr Asp Ile
               20                  25                  30
   Lys
<210> 9
   <211> 40
   <212> PRT
   <213> Human immunodeficiency virus type 2
<400> 9
   Lys Leu Lys Asp Phe Arg Val Tyr Phe Arg Glu Gly Arg Asp Gln Leu
   1               5                   10                  15
   Trp Lys Gly Pro Gly Glu Leu Leu Trp Lys Gly Glu Gly Ala Val Leu
               20                  25                  30
   Val Lys Val Gly Thr Asp Ile Lys
           35                  40
<210> 10
   <211> 33
   <212> PRT
   <213> Human immunodeficiency virus type 2
<400> 10
   Lys Gly Pro Gly Glu Leu Leu Trp Lys Gly Glu Gly Ala Val Leu Val
   1               5                   10                  15
   Lys Val Gly Thr Asp Ile Lys Ile Ile Pro Arg Arg Lys Ala Lys Ile
               20                  25                  30
   Ile
<210> 11
   <211> 36
   <212> PRT
   <213> Human immunodeficiency virus type 2
<400> 11
   Lys Leu Lys Asp Phe Arg Val Tyr Phe Arg Glu Gly Arg Asp Gln Leu
   1 5 10 15
   Trp Lys Gly Pro Gly Glu Leu Leu Trp Lys Gly Glu Gly Ala Val Leu
   20 25 30
   Val Lys Val Gly
   35
<210> 12
   <211> 591
   <212> DNA
   <213> Human immunodeficiency virus type 1
<400> 12
   ccagggatta gatatcagta caatgtgctt ccacagggat ggaaaggatc accagcaata 60
   ttccaaagta gcatgacaaa aatcttagag ccttttagaa aacaaaatcc agacatagtt 120
   atctatcaat acatggatga tttgtatgta ggatctgact tagaaatagg gcagcataga 180
   acaaaaatag aggagctgag acaacatctg ttgaggtggg gacttaccac accagacaaa 240
   aaacatcaga aagaacctcc attcctttgg atgggttatg aactccatcc tgataaatgg 300
   acagtacagc ctatagtgct gccagaaaaa gacagctgga ctgtcaatga catacagaag 360
   ttagtgggaa aattgaattg ggcaagtcag atttacccag ggattaaagt aaggcaatta 420
   tgtaaactcc ttagaggaac caaagcacta acagaagtaa taccactaac agaagaagca 480
   gagctagaac tggcagaaaa cagagagatt ctaaaagaac cagtacatgg agtgtattat 540
   gacccatcaa aagacttaat agcagaaata cagaagcagg ggcaaggcca a 591
<210> 13
   <211> 197
   <212> PRT
   <213> Human immunodeficiency virus type 1
<400> 13
   Pro Gly Ile Arg Tyr Gln Tyr Asn Val Leu Pro Gln Gly Trp Lys Gly
    1               5                  10                  15
   Ser Pro Ala Ile Phe Gln Ser Ser Met Thr Lys Ile Leu Glu Pro Phe
               20                  25                  30
   Arg Lys Gln Asn Pro Asp Ile Val Ile Tyr Gln Tyr Met Asp Asp Leu
           35                  40                  45
   Tyr Val Gly Ser Asp Leu Glu Ile Gly Gln His Arg Thr Lys Ile Glu
       50                  55                  60
   Glu Leu Arg Gln His Leu Leu Arg Trp Gly Leu Thr Thr Pro Asp Lys
   65                  70                  75                  80
   Lys His Gln Lys Glu Pro Pro Phe Leu Trp Met Gly Tyr Glu Leu His
                   85                  90                  95
   Pro Asp Lys Trp Thr Val Gln Pro Ile Val Leu Pro Glu Lys Asp Ser
               100                 105                 110
   Trp Thr Val Asn Asp Ile Gln Lys Leu Val Gly Lys Leu Asn Trp Ala
           115                 120                 125
   Ser Gln Ile Tyr Pro Gly Ile Lys Val Arg Gln Leu Cys Lys Leu Leu
       130                 135                 140
   Arg Gly Thr Lys Ala Leu Thr Glu Val Ile Pro Leu Thr Glu Glu Ala
   145                 150                 155                 160
   Glu Leu Glu Leu Ala Glu Asn Arg Glu Ile Leu Lys Glu Pro Val His
                   165                 170                 175
   Gly Val Tyr Tyr Asp Pro Ser Lys Asp Leu Ile Ala Glu Ile Gln Lys
               180                 185                 190
   Gln Gly Gln Gly Gln
           195
<210> 14
   <211> 258
   <212> DNA
   <213> Human immunodeficiency virus type 1
<400> 14
   tacagtgcag gggaaagaat agtagacata atagcaacag acatacaaac taaagaatta 60
   caaaaacaaa ttacaaaaat tcaaaatttt cgggtttatt acagggacag cagagatcca 120
   ctttggaaag gaccagcaaa gctcctctgg aaaggtgaag gggcagtagt aatacaagat 180
   aatagtgaca taaaagtagt gccaagaaga aaagcaaaga tcattaggga ttatggaaaa 240
   cagatggcag gtgatgat                                               258
<210> 15
   <211> 86
   <212> PRT
   <213> Human immunodeficiency virus type 1
<400> 15
   Tyr Ser Ala Gly Glu Arg Ile Val Asp Ile Ile Ala Thr Asp Ile Gln
   1               5                   10                  15
   Thr Lys Glu Leu Gln Lys Gln Ile Thr Lys Ile Gln Asn Phe Arg Val
               20                  25                  30
   Tyr Tyr Arg Asp Ser Arg Asp Pro Leu Trp Lys Gly Pro Ala Lys Leu
           35                  40                  45
   Leu Trp Lys Gly Glu Gly Ala Val Val Ile Gln Asp Asn Ser Asp Ile
       50                  55                  60
   Lys Val Val Pro Arg Arg Lys Ala Lys Ile Ile Arg Asp Tyr Gly Lys
   65                  70                  75                  80
   Gln Met Ala Gly Asp Asp
                   85
<210> 16
   <211> 40
   <212> DNA
   <213> Artificial Sequence
<220>
   <223> Description of Artificial Sequence: PCR Primer
<400> 16
   agcaccatgg ggatcccagg gattagatat cagtacaatg 40
<210> 17
   <211> 30
   <212> DNA
   <213> Artificial Sequence
<220>
   <223> Description of Artificial Sequence: PCR Primer
<400> 17
   agtcagaatt cattggcctt gcccctgctt 30
<210> 18
   <211> 39
   <212> DNA
   <213> Artificial Sequence
<220>
   <223> Description of Artificial Sequence: PCR Primer
<400> 18
   agcaccatgg ggatccccta cagtgcaggg gaaagaata 39
<210> 19
   <211> 32
   <212> DNA
   <213> Artificial Sequence
<220>
   <223> Description of Artificial Sequence: PCR Primer
<400> 19
   gactagtcga ctcaatcatc acctgccatc tg 32